# EUROPEAN PATENT APPLICATION

(11) **EP 4 851 103 A2**
(43) Date of publication of application: **09.12.2026**
(21) Application number: 26214595.6
(22) Date of filing: 21.03.2023
(51) Int. Cl.: B82Y 20/00

(54) **INTRAOCULAR LENSES WITH NANOSTRUCTURES AND METHODS OF FABRICATING THE SAME**

(30) Priority: 22.03.2022 US 202263269719 P; 29.04.2022 US 202263363828 P; 17.05.2022 US 202263364813 P
(62) Document number(s) of the earlier application(s) in accordance with Art. 76 EPC: 23716671.5 / 4 496 532
(71) Applicant: Alcon Inc., 1701 Fribourg (CH)
(72) Inventor: ZHANG, Hao, Fort Worth, TX 76134 (US); HONG, Xin, Fort Worth, TX 76134 (US); PALIWAL, Sumit, Fort Worth, TX 76134 (US)
(74) Representative: Murgitroyd & Company

(57) **Abstract**

Certain embodiments provide an intraocular lens (IOL) including a lens body having a monolithic anterior lens element having an anterior nanostructure assembly formed thereon and a monolithic posterior lens element having a posterior nanostructure assembly formed thereon, and one or more haptics coupled to the lens body.

## Description

### BACKGROUND

The human eye in its simplest terms functions to provide vision by transmitting light through a clear outer portion called the cornea, and focusing the image by way of a lens onto a retina. The quality of the focused image depends on many factors including the size and shape of the eye, and the transparency of the cornea and lens. When age or disease causes the lens to become less transparent, vision deteriorates because of the diminished light which can be transmitted to the retina. This deficiency in the lens of the eye is medically known as a cataract. An accepted treatment for this condition is surgical removal of the lens and replacement of the lens function by an intraocular lenses (IOLs).

Although existing IOLs as well as methods and systems for manufacturing thereof may be acceptable, they also have certain shortcomings. Accordingly, there is a need for improvements to IOL designs and associated manufacturing techniques for complex optical designs.

### SUMMARY

Aspects of the present disclosure provide an intraocular lens (IOL) including a lens body having a monolithic anterior lens element having an anterior nanostructure assembly formed thereon and a monolithic posterior lens element having a posterior nanostructure assembly formed thereon, and one or more haptics coupled to the lens body.

Aspects of the present disclosure also provide a method for fabricating an intraocular lens (IOL). The method includes fabricating a monolithic anterior lens element and a monolithic posterior lens element, bonding the monolithic anterior lens element and the monolithic posterior lens element to form a cavity therebetweeen, and filling the cavity with an optical fluid. The monolithic anterior lens element has an anterior nanostructure assembly formed thereon, and the monolithic posterior lens element has a posterior nanostructure assembly formed thereon.

Aspects of the present disclosure further provide a method for configuring an intraocular lens (IOL). The method includes computing configurations of an anterior nanostructure assembly formed on an anterior lens element of an IOL and a posterior nanostructure assembly formed on a posterior lens element of the IOL, based on a lens base poser and a desired value of refractive index of the IOL, and forming the IOL or causing the IOL to be formed based on the computed configurations of the anterior nanostructure assembly and the posterior nanostructure assembly.

### BRIEF DESCRIPTION OF THE DRAWINGS

So that the manner in which the above recited features of the present disclosure can be understood in detail, a more particular description of the disclosure, briefly summarized above, may be had by reference to embodiments, some of which are illustrated in the appended drawings. It is noted, however, that the appended drawings illustrate only some aspects of this disclosure and the disclosure may admit to other equally effective embodiments.
Figure 1A depicts a top view of an intraocular lens (IOL), according to certain embodiments.
Figure 1B depicts a side view of a portion of the IOL of Figure 1A, according to certain embodiments.
Figure 1C depicts an enlarged view of a portion of the IOL of Figure 1A, according to certain embodiments.
Figure 2 depicts example operations for fabricating an IOL with nanostructures, according to certain embodiments.
Figure 3 depicts example operations for forming a lens element, according to certain embodiments.
Figures 4A, 4B, and 4C illustrate various aspects of a lens element corresponding to the different stages of the operations of Figure 3, according to certain embodiments.
Figure 5 depicts an example system for designing, configuring, and/or forming an IOL, according to certain embodiments.
Figure 6 depicts example operations for forming an IOL, according to certain embodiments.

To facilitate understanding, identical reference numerals have been used, where possible, to designate identical elements that are common to the figures. It is contemplated that elements and features of one embodiment may be beneficially incorporated in other embodiments without further recitation.

### DETAILED DESCRIPTION

The embodiments described herein provide methods and systems for fabricating an intraocular lens (IOL) having nanostructured patterns embossed onto on its external surfaces of the IOL. In certain embodiments, the methods and the system include producing an anterior lens element and a posterior lens element, each having nanostructured patterns, bonding the anterior and posterior lens elements to form a cavity therebetween, and filling the cavity with an optical fluid.

### An IOL with Nanostructures

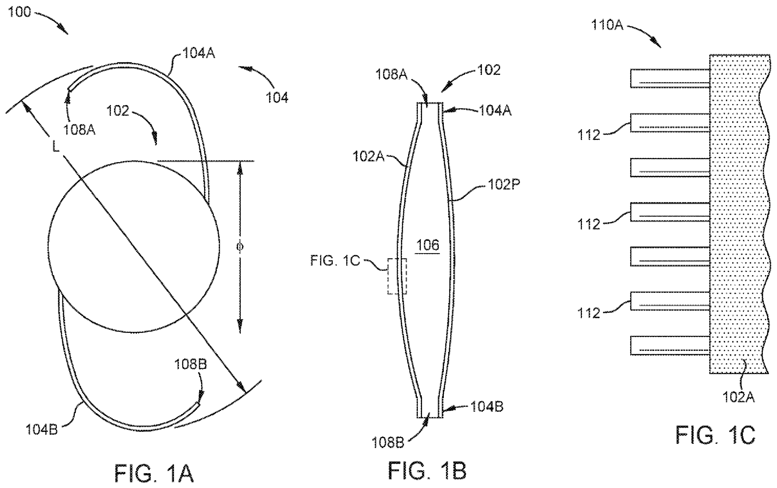

Figure 1A illustrates a top view of an intraocular lens (IOL) 100, according to certain embodiments. Figure 1B illustrates a side view of a portion of the IOL 100. FIG. 1C illustrates an enlarged side view of a portion of the IOL 100. The IOL 100 includes a lens body 102 and a haptic portion 104 that is coupled to a peripheral, non-optic portion of the lens body 102.

The lens body 102 includes an anterior lens element 102A and a posterior lens element 102P. The lens elements 102A and 102P are bonded together to form a cavity 106. The cavity 106 is filled with an optical fluid. The optical fluid may be an incompressible or substantially incompressible fluid exhibiting a refractive index that is different from the lens elements 102A and 102P. The optical fluid may be a refractive index-matched silicone oil of ophthalmic grade, such as the optical fluid available from Entegris, Inc., Billerica, Massachusetts. The anterior lens element 102A and the posterior lens element 102P may be fabricated of a transparent, flexible, biocompatible polymer, such as flexible polymer, including poly (dimethylsiloxane) (PDMS). The lens body 102 has a diameter *ϕ* of between about 4.5 mm and about 7.5 mm, for example, about 6.0 mm.

The haptic portion 104 includes hollow radially-extending struts (also referred to as "haptics") 104A and 104B that are coupled (*e.g.,* glued or welded) to the peripheral portion of the lens body 102 or molded along with a portion of the lens body 102, and thus extend outwardly from the lens body 102 to engage the perimeter wall of the capsular sac of the eye to maintain the lens body 102 in a desired position in the eye. The haptics 104A and 104B each have an internal volume 108A, 108B, which is in fluid communication with the cavity 106 of the lens body 102. The haptics 104A and 104B may be fabricated of biocompatible material, such as modified poly (methyl methacrylate) (PMMA), modified PMMA hydrogels, hydroxy-ethyl methacrylate (HEMA), PVA hydrogels, other silicone polymeric materials, hydrophobic acrylic polymeric materials, for example, AcrySof^{®} and Clareon^{®}, available from Alcon, Inc., Fort Worth, Texas. The haptics 104A and 104B typically have radial-outward ends that define arcuate terminal portions. The terminal portions of the haptics 104A and 104B may be separated by a length *L* of between about 6 mm and about 22 mm, for example, about 13 mm. The haptics 104A and 104B have a particular length so that the terminal portions create a slight engagement pressure when in contact with the equatorial region of the capsular sac after being implanted. While Figure 1A illustrates one example configuration of the haptics 104A and 104B, any plate haptics or other types of haptics can be used.

It is noted that the shape and curvatures of the lens body 102 are shown for illustrative purposes only and that other shapes and curvatures are also within the scope of this disclosure. For example, the lens body 102 shown in Figure 1B has a bi-convex shape. In other examples, the lens body 102 may have a plano-convex shape, a convexo-concave shape, or a plano-concave shape. In certain embodiments, as shown in Figure 1B, the IOL 100 is a mono-focal IOL (with a single focal point) having no annular echelettes formed on the anterior lens element 102A or the posterior lens element 102P. In some other embodiments, the IOL 100 is a multi-focal IOL (with multiple focal points, *e.g.,* bi-focal and tri-focal) having annular echelettes on the anterior lens element 102A and/or the posterior lens element 102P (not shown). In some other embodiments, the IOL 100 is an extended depth of focus (EDOF) IOL (with elongated focus) having annular echelettes on the posterior lens element 102P (not shown).

In the embodiments described herein, the anterior lens element 102A includes an anterior nanostructure assembly 110A formed thereon, and the posterior lens element 102P includes a posterior nanostructure assembly 110P formed thereon (not shown). The anterior nanostructure assembly 110A and the anterior lens element 102A are formed as a monolithic single piece of the same material, such as flexible polymer, including PDMS. The posterior nanostructure assembly 110P and the posterior lens element 102P are also formed as a monolithic single piece of the material, such as flexible polymer, including PDMS. In certain embodiments, the lens body 102 includes only one of the anterior nanostructure assembly 110A or the posterior nanostructure assembly 110P. The lens element 102A, or 102P that does not have a nanostructure assembly formed thereon may include a diffractive structure to adjust a refractive index and/or reflectivity of the lens.

As shown in Figure 1C, the anterior nanostructure assembly 110A includes protrusions 112. The posterior nanostructure assembly 110P includes similar protrusions 112. Shapes, sizes, and density (*e.g.,* spacing between adjacent protrusions 112) of the protrusions 112 are designed for the lens body 102 to provide a desired refractive index. For example, the protrusions 112 may have heights *H* of between about 30 nm and about 200 nm, widths of between about 30 nm and about 300 nm, and spacings between adjacent protrusions 112 of between about 30 nm and about 300 nm. The nanostructure assemblies 110A, 110P may further reduce reflectivity by between about 10 % and about 90 %, as compared to a lens body having no nanostructure assemblies on its anterior outer surface or posterior outer surface. In certain embodiments, as shown in Figure 1B, the nanostructure assemblies 110A, 110P entirely cover the anterior lens element 102A and the posterior lens element 102P, respectively. However, in some other embodiments, the nanostructure assemblies 110A, 110P only partially cover the anterior lens element 102A and the posterior lens element 102P, respectively.

### Fabrication of an IOL with Nanostructures

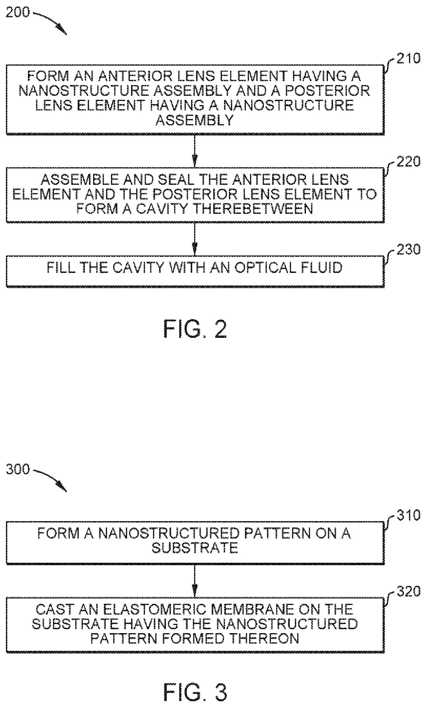

Figure 2 depicts example operations 200 for forming an IOL with nanostructures, according to certain embodiments.

At step 210, an anterior lens element *(e.g.,* anterior lens element 102A) having a nanostructure assembly (*e.g.,* nanostructure assembly 110A (shown in Figure 1C)) and a posterior lens element (*e.g.,* posterior lens element 102P) having a nanostructure assembly (*e.g.,* nanostructure assembly 110P) are formed, as described below in relation to operations 300.

At step 220, the anterior lens element and the posterior lens element are assembled and sealed to form a cavity (*e.g.,* cavity 106 (shown in Figure 1B)). The anterior lens element and the posterior lens element may be bonded together to make a seal in a peripheral non-optic portion of the lens body (*e.g.,* lens body 102), by chemical bonding, thermal bonding, UV bonding or other appropriate types of bonding, with the cavity therebetween.

At step 230, the cavity is filled with an optical fluid, like silicone oil.

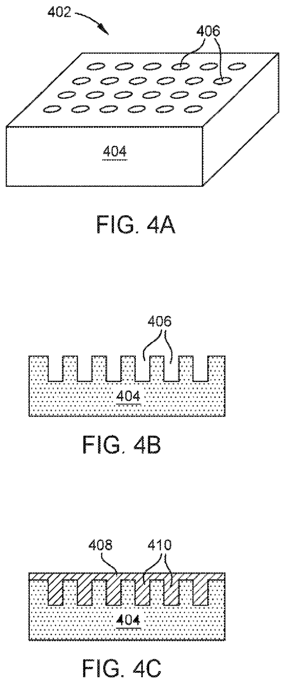

Figure 3 depicts example operations 300 for forming each of the lens elements 102A, 102P (*i.e.,* step 210). Figures 4A, 4B, and 4C illustrate various aspects of a lens element corresponding to the different stages of the operations 300. As such, Figures 3, 4A, 4B, and 4C are described together.

At step 310, a nanostructured pattern (*e.g.,* nanostructured pattern 402) is formed on a substrate (*e.g.,* substrate 404) by standard micro/nano fabrication methods, as shown in an isometric view in Figure 4A and a cross-sectional view in Figure 4B. For example, first, a photolithography process is performed to spin on a photoresist on the substrate, and selected areas of the photoresist are exposed to light and developed. Second, the pattern of the photoresist is transferred to the substrate by reactive ion etching. Then, the remaining photoresist is removed by plasma over-etching. The nanostructured pattern may extend over an area of about 7 mm and about 7 mm on the substrate, and include an array of trenches (*e.g.,* array of trenches 406) carved in the substrate with heights of between about 30 nm and about 200 nm, widths of between about 30 nm and about 300 nm, and spacings between adjacent trenches of between about 30 nm and about 300 nm.

The term "substrate" as used herein refers to a layer of material that serves as a basis for subsequent processing operations and includes a surface to be cleaned. For example, the substrate may include glass, or one or more conductive metals, such as nickel, titanium, platinum, molybdenum, rhenium, osmium, chromium, iron, aluminum, copper, tungsten, or combinations thereof. The substrate can also include one or more materials comprising silicon, including materials associated with group IV or group III-V including compounds, such as Si, polysilicon, amorphous silicon, silicon nitride, silicon oxynitride, silicon oxide, Ge, SiGe, GaAs, InP, InAs, GaAs, GaP, InGaAs, InGaAsP, GaSb, InSb and the like, or combinations thereof. Furthermore, the substrate can also include dielectric materials such as silicon dioxide, organosilicates, and carbon doped silicon oxides. Further, the substrate can include any other materials such as metal nitrides, metal oxides and metal alloys, depending on the application.

Moreover, the substrate is not limited to any particular size or shape. The substrate can be a round wafer having a 200 mm diameter, a 300 mm diameter, a 450 mm diameter or other diameters. The substrate can also be any polygonal, square, rectangular, curved or otherwise non-circular workpiece, such as a polygonal glass, plastic substrate.

At step 320, an elastomeric membrane (e.g., elastomeric membrane 408) is casted on the substrate having the nanostructured pattern formed thereon, as shown in exemplary Figure 4C. The elastomeric membrane may be formed of a thin PDMS. In the casting process, PDMS in a liquid form may be mixed with a cross-linking agent and poured onto the substrate and heated at an elevated temperature of between about 250 °C and about 350 °C, hardening and cross-linking PDMS, to form the elastomeric membrane. The elastomeric membrane may have a thickness of between about 200 µm and 500 µm. The elastomeric membrane has protrusions (*e.g.,* protrusions 410) that replicate the nanostructured pattern on the substrate. The protrusions may have heights of between about 30 nm and about 200 nm, widths of between about 30 nm and about 300 nm, and spacings between adjacent trenches of between about 30 nm and about 300 nm. After casting, the elastomeric membrane is removed from the substrate. This elastomeric membrane can be used as the anterior lens element having the anterior nanostructure assembly or the posterior lens element having the posterior nanostructure assembly (shown in exemplary Figure 1C).

### System for Designing an IOL

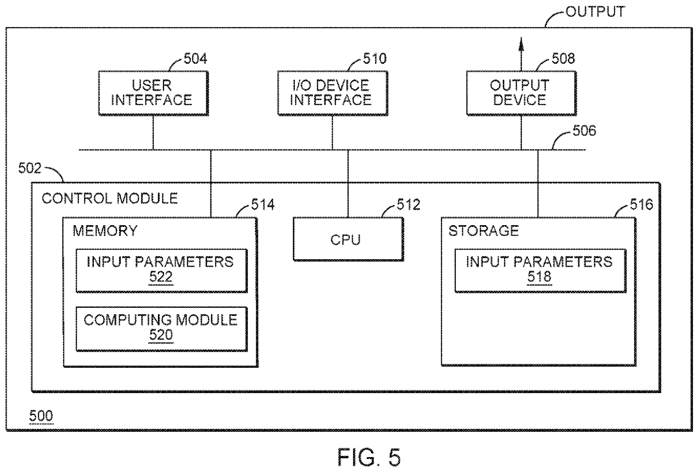

Figure 5 depicts an exemplary system 500 for designing, configuring, and/or forming an IOL 100. As shown, the system 500 includes, without limitation, a control module 502, a user interface display 504, an interconnect 506, an output device 508, and at least one I/O device interface 510, which may allow for the connection of various I/O devices (e.g., keyboards, displays, mouse devices, pen input, etc.) to the system 500.

The control module 502 includes a central processing unit (CPU) 512, a memory 514, and a storage 516. The CPU 512 may retrieve and execute programming instructions stored in the memory 514. Similarly, the CPU 512 may retrieve and store application data residing in the memory 514. The interconnect 506 transmits programming instructions and application data, among CPU 512, the I/O device interface 510, the user interface display 504, the memory 514, the storage 516, output device 508, etc. The CPU 512 can represent a single CPU, multiple CPUs, a single CPU having multiple processing cores, and the like. Additionally, in certain embodiments, the memory 514 represents volatile memory, such as random access memory. Furthermore, in certain embodiments, the storage 516 may be non-volatile memory, such as a disk drive, solid state drive, or a collection of storage devices distributed across multiple storage systems.

As shown, the storage 516 includes input parameters 518. The input parameters 518 include a lens base power and a desired value of refractive index of a lens body. The memory 514 includes a computing module 520 for computing control parameters, such as configuration of the nanostructure assemblies 110A, 110P (*e.g.,* shapes, sizes, and density). In addition, the memory 514 includes input parameters 522.

In certain embodiments, input parameters 522 correspond to input parameters 518 or at least a subset thereof. In such embodiments, during the computation of the control parameters, the input parameters 522 are retrieved from the storage 516 and executed in the memory 514. In such an example, the computing module 520 comprises executable instructions (*e.g.,* including one or more of the formulas described herein) for computing the control parameters, based on the input parameters 522. In certain other embodiments, input parameters 522 correspond to parameters received from a user through user interface display 504. In such embodiments, the computing module 520 comprises executable instructions for computing the control parameters, based on information received from the user interface display 504.

In certain embodiments, the computed control parameters, are output via the output device 508 to a lens manufacturing system that is configured to receive the control parameters and form a lens accordingly. In certain other embodiments, the system 500 itself is representative of at least a part of a lens manufacturing systems. In such embodiments, the control module 502 then causes hardware components (not shown) of system 500 to form the lens according to the control parameters by the operations 200 described above.

### Method for Forming an IOL

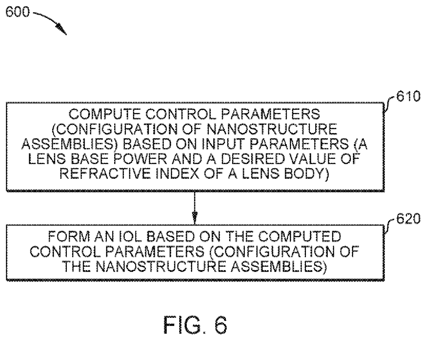

Figure 6 depicts example operations 600 for forming an IOL (*e.g.,* IOL 100). In some embodiments, the step 610 of operations 600 is performed by one system (*e.g.,* the system 500) while step 620 is performed by a lens manufacturing system. In some other embodiments, both steps 610 and 620 are performed by a lens manufacturing system.

At step 610, control parameters, such as configuration of the nanostructure assemblies 110A, 110P (*e.g.,* shapes, sizes, and density) are computed based on input parameters (*e.g.,* a lens base power and a desired value of refractive index of a lens body). The computations performed at step 610 are based on one or more of the embodiments, including the formulas, described herein.

At step 620, an IOL (*e.g.,* IOL 100) based on the computed control parameters, such as configuration of the nanostructure assemblies 110A, 110P (*e.g.,* shapes, sizes, and density) is formed according to the operations 200 described above, using appropriate methods, systems, and devices typically used for manufacturing lenses, as known to one of ordinary skill in the art.

The embodiments described herein provide methods and systems for fabricating an IOL having nanostructured patterns embossed onto the external surfaces of the IOL, by producing an anterior lens element and a posterior lens element, each having nanostructured patterns, bonding the anterior and posterior lens elements to form a cavity therebetween, and filling the cavity with an optical fluid. The methods described herein may offer simplified fabrication processes of IOLs with nanostructured patterns embossed on external surfaces as compared to the conventional fabrication processes. Additionally, the described methods may further eliminate concerns associated with disintegration of implanted IOLs that were formed by separately fabricating nanostructures which are subsequently attached to the IOL. Therefore, the improved manufacturing techniques disclosed herein allow for manufacturing improved IOLs with nanostructures on the external surfaces for reducing customer complaints about glare and halo, as well as issues with reflection possibly associated with the scary eye phenomena.

While the foregoing is directed to embodiments of the present disclosure, other and further embodiments of the disclosure may be devised without departing from the basic scope thereof, and the scope thereof is determined by the claims that follow.
This application is a divisional application based on EP 23716671.5 (based on PCT/IB2023/052774 WO 2023/180934) the entire contents (specification and drawings) of which are herein incorporated by reference. This divisional having the same priorities as the parent application. Further arrangements according to the specification (based on the claims of the parent patent application as filed) are set out in the following numbered clauses.

### CLAUSES

1. An intraocular lens (IOL), comprising: a lens body having a monolithic anterior lens element having an anterior nanostructure assembly formed thereon and a monolithic posterior lens element having a posterior nanostructure assembly formed thereon; and one or more haptics coupled to the lens body.
2. The IOL of clause 1, wherein the monolithic anterior lens element and the monolithic posterior lens element each comprises poly (dimethylsiloxane) (PDMS).
3. The IOL of clause 1, further comprising: an optical fluid filled in a cavity formed between the monolithic anterior lens element and the monolithic posterior lens element.
4. The IOL of clause 3, wherein the optical fluid comprises silicone oil.
5. The IOL of clause 3, wherein the one or more haptics each include an internal volume that is in fluid communication with the cavity.
6. The IOL of clause 1, wherein each of the anterior nanostructure assembly and the posterior nanostructure assembly includes a plurality of protrusions having heights of between 30 nm and 200 nm, widths of between 30 nm and 300 nm, and spacings between adjacent protrusions of between 30 nm and 300 nm.
7. The IOL of clause 1, wherein the anterior nanostructure assembly and the posterior nanostructure assembly are configured to reduce reflectivity of the lens body by between 10 % and 90 % as compared to a lens body with no nanostructure assemblies on the monolithic anterior lens element or the monolithic posterior lens element.
8. A method for fabricating an intraocular lens (IOL), comprising:
   fabricating a monolithic anterior lens element and a monolithic posterior lens element, wherein the monolithic anterior lens element has an anterior nanostructure assembly formed thereon, and
   the monolithic posterior lens element has a posterior nanostructure assembly formed thereon; bonding the monolithic anterior lens element and the monolithic posterior lens element to form a cavity therebetweeen; and filling the cavity with an optical fluid.
9. The method of clause 8, wherein the fabricating of each of the monolithic anterior lens element and the monolithic posterior lens element comprises: casting an elastomeric membrane on a substrate having a nanostructured pattern formed thereon; and removing the elastomeric membrane from the substrate.
10. The method of clause 9, wherein the elastomeric membrane comprises poly (dimethylsiloxane) (PDMS).
11. The method of clause 9, wherein the nanostructured pattern formed on the substrate extends over an area of 7 mm and 7 mm, and includes an array of trenches with heights of between 30 nm and 200 nm, widths of between 30 nm and 300 nm, and spacings between adjacent trenches of between 30 nm and 300 nm.
12. The method of clause 8, wherein the anterior nanostructure assembly and the posterior nanostructure assembly each include a plurality of protrusions having heights of between 30 nm and 200 nm, widths of between 30 nm and 300 nm, and spacings between adjacent trenches of between 30 nm and 300 nm.
13. The method of clause 8, wherein the optical fluid comprises silicone oil.
14. The method of clause 8, further comprising: attaching a haptic portion to a peripheral non-optic portion of the monolithic anterior lens element and the monolithic posterior lens element,
   wherein the haptic portion comprises one or more hollow haptics having an internal volume that is in fluid communication with the cavity.
15. A method for configuring an intraocular lens (IOL), comprising:
   computing configurations of an anterior nanostructure assembly formed on an anterior lens element of an IOL and a posterior nanostructure assembly formed on a posterior lens element of the IOL, based on a lens base power and a desired value of refractive index of the IOL; and
   forming the IOL or causing the IOL to be formed based on the computed configurations of the anterior nanostructure assembly and the posterior nanostructure assembly.
16. The method of clause 15, wherein the forming of the IOL or the causing of the IOL to be formed comprises: fabricating the anterior lens element and the posterior lens element; bonding the anterior lens element and the posterior lens element to form a cavity therebetween; and filling the cavity with an optical fluid.
17. The method of clause 16, wherein the fabricating of each of the anterior lens element and the posterior lens element comprises: casting an elastomeric membrane a substrate having a nanostructured pattern formed thereon; and removing the elastomeric membrane from the substrate.
18. The method of clause 17, wherein the elastomeric membrane comprises poly (dimethylsiloxane).
19. The method of clause 17, wherein the nanostructured pattern formed on the substrate extends over an area of 7 mm and 7mm, and includes an array of trenches with heights of between 30 nm and 200 nm, widths of between 30 nm and 300 nm, and spacings between adjacent trenches of between 30 nm and 300 nm.
20. The method of clause 16, wherein the optical fluid comprises silicone oil.

## Claims

1. An intraocular lens (IOL), comprising:
a lens body comprising:
a monolithic anterior lens element comprising a cast elastomeric membrane having nanostructured protrusions formed thereon that replicate a first nanostructured substrate pattern formed on a first substrate; and
a monolithic posterior lens element comprising a cast elastomeric membrane having nanostructured protrusions formed thereon that replicate a second nanostructured substrate pattern formed on a second substrate; and
one or more haptics coupled to the lens body.

2. The IOL of claim 1, wherein the first nanostructured substrate pattern and the second nanostructured substrate pattern each comprise a respective array of trenches.

3. The IOL of claim 2, wherein the trenches of each respective array have heights of between 30 nm and 200 nm, widths of between 30 nm and 300 nm, and spacings between adjacent trenches of between 30 nm and 300 nm.

4. The IOL of claim 1, wherein the nanostructured protrusions of each of the monolithic anterior lens element and the monolithic posterior lens element have heights of between 30 nm and 200 nm, widths of between 30 nm and 300 nm, and spacings between adjacent nanostructured protrusions of between 30 nm and 300 nm.

5. The IOL of claim 1, wherein respective configurations of the nanostructured protrusions of the monolithic anterior lens element and the monolithic posterior lens element are defined by shapes, sizes, and densities of the nanostructured protrusions.

6. The IOL of claim 5, wherein the respective configurations of the nanostructured protrusions are configured to provide a refractive index of the lens body.

7. The IOL of claim 1, wherein each cast elastomeric membrane comprises poly(dimethylsiloxane) (PDMS).

8. The IOL of claim 1, wherein each cast elastomeric membrane has a thickness of between 200 µm and 500 µm.

9. The IOL of claim 1, wherein the nanostructured protrusions entirely cover respective external surfaces of the monolithic anterior lens element and the monolithic posterior lens element.

10. The IOL of claim 1, wherein the nanostructured protrusions reduce reflectivity of the lens body by between 10% and 90% as compared to a lens body without the nanostructured protrusions.

11. The IOL of claim 1, further comprising an optical fluid filled in a cavity formed between the monolithic anterior lens element and the monolithic posterior lens element.

12. The IOL of claim 11, wherein the optical fluid comprises silicone oil and has a refractive index different from a refractive index of the monolithic anterior lens element and the monolithic posterior lens element.

13. The IOL of claim 11, wherein the one or more haptics each comprise an internal volume in fluid communication with the cavity.

14. A method for fabricating a lens element for an intraocular lens (IOL), comprising:
casting an elastomeric membrane on a substrate having a nanostructured pattern formed thereon, wherein the elastomeric membrane includes a plurality of protrusions that replicate the nanostructured pattern; and
removing the elastomeric membrane from the substrate,
wherein the elastomeric membrane constitutes the lens element.

15. The method of claim 14, wherein casting the elastomeric membrane comprises:
mixing poly(dimethylsiloxane) (PDMS) in liquid form with a cross-linking agent;
pouring the PDMS mixed with the cross-linking agent onto the substrate; and
heating the PDMS mixed with the cross-linking agent at a temperature of between 250 °C and 350 °C to harden and cross-link the PDMS to form the elastomeric membrane.
